# EUROPEAN PATENT SPECIFICATION

(11) **EP 2 815 752 B1**
(45) Date of publication and mention of the grant of the patent: **20.05.2020**
(21) Application number: 13749905.9
(22) Date of filing: 14.02.2013
(51) Int. Cl.: A61K 31/513, A61K 31/7072, A61K 9/00, A61K 9/20, A61P 35/00

(54) **ORAL PHARMACEUTICAL COMPOSITION**
ORALE PHARMAZEUTISCHE ZUSAMMENSETZUNG
COMPOSITION PHARMACEUTIQUE ORALE

(30) Priority: 15.02.2012 JP 2012031144
(43) Date of publication of application: 24.12.2014
(73) Proprietor: Taiho Pharmaceutical Co., Ltd., Chiyoda-ku Tokyo 101-8444 (JP)
(72) Inventor: OHNISHI, Yoshito, Tokushima-shi Tokushima 771-0194 (JP); OGATA, Tetsuo, Tokushima-shi Tokushima 771-0194 (JP)
(74) Representative: Blodig, Wolfgang
(86) International application number: PCT/JP2013/053514
(87) International publication number: WO 2013/122135

(56) References cited:
- EP-A1- 0 763 529
- EP-A1- 1 230 925
- EP-A1- 1 849 470
- WO-A1-96/30346
- WO-A1-2006/080327
- JP-A- H11 322 604
- JP-A- 2001 131 075
- JP-A- 2005 112 744
- JP-A- 2005 314 413
- JP-A- 2007 284 390

## Description

### [Technical Field]

The present disclosure relates to an oral pharmaceutical composition comprising α,α,α-trifluorothymidine (FTD) and 5-chloro-6-(2-iminopyrrolidine-1-yl)methyl-2,4(1H,3H)-pyrimidine dione hydrochloride (TPI).

### [Background Art]

A combination drug comprising α,α,α-trifluorothymidine (FTD) and 5-chloro-6-(2-iminopyrrolidine-1-yl)methyl-2,4(1H,3H)-pyrimidine dione hydrochloride (TPI) is an anti-tumor agent in which FTD, which has an action for inhibiting thymidylate formation and an action for inhibiting DNA synthesis by incorporation into DNA to exert an anti-tumor effect, is combined with TPI, which has an action for inhibiting thymidine phosphorylase, to thereby suppress degradation of FTD in vivo and enhance the anti-tumor effect (Patent Literature 1).

An anti-tumor agent "TAS-102" in which FTD and TPI are combined in a molar ratio of 1:0.5 is now under development as an orally administrable formulation (Non Patent Literatures 1 and 2). As for the orally-administrable TAS-102 formulation, tablets, granules, capsules, and the like are known so far (Patent Literatures 1, 2 and 3). However, the quality, particularly the storage stability of the formulation has not been sufficiently investigated.

Meanwhile, in medical settings, in order to prevent accidental ingestion and to enhance medication compliance, one-dose packaging to package various medicaments into each one dosage form is promoted, and thus, stable and high-quality formulations are desired even without moisture-proof packaging. Alternatively, even in the case that moisture-proof packaging is applied, pharmaceutical formulation has to be devised in order to suppress deterioration in quality due to moisture absorption after opening.

### [Citation List]

### [Patent Literature]

[Patent Literature 1]
   International Publication No. WO 96/30346
[Patent Literature 2]
   International Publication No. WO 2006/80327
[Patent Literature 3]
   EP 1 230 925 A1

### [Non Patent Literature]

[Non Patent Literature 1]
   International Journal of Oncology 25: 571-578, 2004
[Non Patent Literature 2]
   Invest New Drugs 26(5): 445-54, Oct 2008.

### [Summary of Invention]

### [Technical Problem]

The present inventor has added various formulation additives to the above FTD and TPI, and has investigated the storage stability of the resulting compositions under various conditions. Then, it has been proved that the amount of FTD and TPI related substances were increased when stored particularly under high-humidity conditions depending on types of formulation additives added.

Accordingly, an object of the present invention is to provide an FTD and TPI-containing oral pharmaceutical composition which can be orally administered and whose active ingredients are stable even under high-humidity conditions.

### [Solution to Problem]

Thus, the present inventor has mixed various additives to FTD and TPI and evaluated the storage stability, and has found that, since mass of related substances is increased when a composition in which a metal salt is employed as an additive is stored, a stable oral pharmaceutical composition can be obtained as long as it is free of the metal salts, completing the present invention.

That is, the present invention relates to the subject matter of claims 1 to 13. In particular, the present invention provides an oral pharmaceutical composition, which is an uncoated tablet, comprising FTD and TPI as active ingredients; comprising a sugar alcohol or a disaccharide as an excipient, one or more selected from the group consisting of hydrogenated oils and stearic acid as a lubricant; and being free of additives comprising a metal salt.

Also, the present invention provides an oral pharmaceutical formulation comprising the above-described oral pharmaceutical composition, which is coated.

### [Advantageous Effects of Invention]

According to the present invention, high-quality formulations can be provided to patients and medical staffs because of secured formulation stability even under high-temperature and/or high-humidity conditions.

### [Description of Embodiments]

The active ingredients of the oral pharmaceutical composition of the present invention are FTD and TPI. The molar ratio of FTD and TPI contained in the composition is preferably 1:0.5. Also, the content of FTD per dosage unit of the oral pharmaceutical composition is preferably from 5 to 35 mg and more preferably from 15 to 20 mg.

Although the contents of FTD and TPI, which are the active ingredients of the oral pharmaceutical composition of the present invention, depend on formulation forms and regimens, and may be selected without particular limitation and as appropriate, the amount of each active ingredient in pharmaceutical composition is preferably from of the order of 1 to 40% by mass.

The oral pharmaceutical composition of the present invention is characterized by being free of metal salts which increase formation of related substances of FTD and TPI. Examples of such metal salts include alkali metal salts and alkaline earth metal salts.

Examples of the alkali metal salts include sodium salts, such as sodium benzoate, sodium alginate, sodium ascorbate, sodium aspartate, sodium hydrogen carbonate, sodium hydrogen sulfite, sodium carbonate, sodium carboxymethyl starch, casein sodium, carmellose sodium, sodium chloride, sodium citrate, sodium citrate anhydride, sodium copper chlorophyllin, sodium dehydroacetate, sodium dihydrogen phosphate, sodium erythorbate, sodium hydroxide, sodium lauryl sulfate, sodium DL-malate, sodium metabisulfite, sodium oleate, sodium polyphosphate, sodium salicylate, monosodium fumarate, sodium sulfite, sodium DL-tartrate, and sodium L-tartrate; and potassium salts, such as carmellose potassium, potassium carbonate, potassium hydrogen tartrate, potassium carbonate, potassium chloride, and potassium sorbate. Examples of the alkaline earth metal include calcium salts, such as calcium acetate, calcium carbonate, calcium chloride, carmellose calcium, calcium citrate, calcium gluconate, calcium lactate, calcium monohydrogen phosphate, calcium silicate, calcium stearate, and calcium sulfate; magnesium salts, such as magnesium aluminometasilicate, magnesium aluminosilicate, magnesium carbonate, magnesium chloride, magnesium hydroxide, alumina magnesium hydroxide, magnesium oxide, magnesium silicate, magnesium aluminosilicate, and magnesium stearate. Examples also include natural products containing alkali metal salts and/or alkaline earth metal salts, such as talc.

Of these metal salts, from a viewpoint of the stability of the active ingredients, it is preferred to be particularly free of alkaline earth metal salts, and furthermore, it is more preferred to be free of talc, carmellose calcium, and magnesium stearate.

In the oral pharmaceutical composition of the present invention, "is free of additives comprising a metal salt" means that additives comprising a metal salt are not contained at all or a small amount of the additives is contained to the extent that the stability of FTD and TPI is not impaired. A specific content of the metal salts is more preferably from 0 to 0.1 parts by mass, more preferably from 0 to 0.05 parts by mass, still more preferably from 0 to 0.01 parts by mass, and particularly preferably 0 parts by mass, based on 1 part by mass of FTD.

The oral pharmaceutical composition of the present invention, which is free of additives comprising a metal salt and contains a specific sugar alcohol or disaccharide as an excipient, fulfills sufficient functions as an oral pharmaceutical composition while suppressing increases in formation of related substances of FTD and TPI even stored under high humidity conditions.

Sugar alcohol or disaccharides which can be employed in the present invention are preferably lactose (including anhydrides and hydrates), sucrose, mannitol or erythritol, more preferably lactose, sucrose, or mannitol, and particularly preferably lactose or mannitol. It should be noted that these sugars may be used singly or in combination of two or more.

The content of the sugar alcohol or disaccharides in the oral pharmaceutical composition of the present invention is, from viewpoints of the stability of FTD and TPI and of the function as an excipient, preferably 3.6 parts by mass or more, more preferably from 3.6 to 50 parts by mass, still more preferably from 3.7 to 25 parts by mass, and still more preferably from 3.7 to 10 parts by mass, based on 1 part by mass of FTD.

The proportion of a sugar alcohol or disaccharide in an oral pharmaceutical composition of the present invention is, from a viewpoint of the stability of the active ingredients, preferably from 50 to 100% by mass, more preferably a range from 70 to 100% by mass, and particularly preferably from 70 to 98% by mass, based on the total amount of the additives.

Alternatively, excipients other than sugar alcohol and disaccharides may be added to the orally administrable pharmaceutical composition of the present invention. From a viewpoint of the stability of the active ingredients, the proportion of sugar alcohol or disaccharides is preferably 50% by mass or more, more preferably 70% by mass or more, more preferably 90% by mass or more, and particularly preferably 100% by mass in the total excipients.

Also, in addition to the above-described excipients, disintegrating agents can be added to the oral pharmaceutical composition of the present invention in order to secure good disintegrability at oral administration. Examples of the disintegrating agent include low-substituted hydroxypropyl cellulose, corn starch, partly pregelatinized starch, carmellose, crospovidone, and crystalline cellulose. From a viewpoint of the stability of FTD and TPI, low-substituted hydroxypropyl cellulose, corn starch, partly pregelatinized starch, or carmellose is preferred, and low-substituted hydroxypropyl cellulose, corn starch, or partly pregelatinized starch is particularly preferred. These may be used singly or in combination of two or more. The content of the disintegrating agent is, from a viewpoint of combining the uniformity of the content of the medicament in the oral pharmaceutical composition of the present invention and the disintegrability of the tablets, preferably from 2 to 16% by mass, more preferably from 3 to 13% by mass, still more preferably from 3 to 10% by mass, and particularly preferably from 3 to 7% by mass in the total amount of the oral pharmaceutical composition.

The oral pharmaceutical composition of the present invention may further contain various additives generally used, to the extent that the effects of the present invention are not prevented. Examples of the additive include, but not particularly limited to, as long as the additive is one generally used, excipients other than the aforementioned sugar, binder, lubricants, colorants, flavoring agents and taste-masking agents (except metal salt).

Examples of the binder include hydroxypropyl cellulose, hypromellose, and polyvinyl alcohol. Examples of the lubricant include hydrogenated oils, stearic acid, and sucrose fatty acid esters. Hydrogenated oils or stearic acid is preferred, and stearic acid is more preferred. Examples of the colorant include food yellow No. 5 dye, food blue No. 2 dye, food lake dyes, ferric oxide, yellow ferric oxide, and titanium oxide. Examples of the flavoring agent include various orange and lemon perfumes. Examples of the taste-masking agent include 1-menthol, camphor, and mint. These may be used singly or in combination of two or more.

The content of the binder herein is preferably from 0.001 to 5% by mass and more preferably from 0.01 to 3% by mass in the total composition. The content of the lubricant is preferably from 0.001 to 3% by mass and more preferably from 0.01 to 2% by mass in the total composition.

The oral pharmaceutical composition of the present invention is an uncoated tablet.

Although the oral pharmaceutical composition of the present invention may be used as it is as a pharmaceutical formulation, the formulation can be further coated on its surface to be an oral pharmaceutical formulation which is stable and easily ingested. Coating herein includes film coating and sugar coating. Examples of a coating base include hypromellose, ethyl cellulose, hydroxypropyl cellulose, polyvinyl alcohol, and sucrose. It should be noted that, in the case of coating an oral pharmaceutical composition containing FTD and TPI stably, the coating layer may contain coatings including metal salt, plasticizers, colorants, flavoring agents, taste-masking agents, and lubricants to the extent that the stability of FTD and TPI is not substantially influenced. Examples of the plasticizer include polyethylene glycol. Examples of the colorant include food tar dyes, food tar dye lakes, ferric oxide, yellow ferric oxide, and titanium oxide. Examples of the flavoring agent include various orange and lemon perfumes. Examples of the taste-masking agent include 1-menthol, camphor, and mint, which may be used singly or in combination of two or more. Additionally, after coating, a lubricant containing metal salts, such as magnesium stearate, talc, and light anhydrous silicic acid can be added on the surface. The total amount of the coating layer herein is preferably from 1 to 5% by mass and more preferably from 2 to 4% by mass in the total formulation.

The oral pharmaceutical formulation of the present disclosure are tablets. Examples of the tablets include chewable tablets, troches, drops, and compositions which quickly dissolve or disintegrate in the mouth cavity and can be ingested even without water, and also include effervescent tablets which are dissolved to be used at time of use.

The oral pharmaceutical composition and pharmaceutical formulation of the present invention can be produced in accordance with the known method for producing orally administrable formulations. Examples of the granulation method include fluid bed granulation methods, stirring granulation methods, tumbling fluid bed granulation methods, extruding granulation methods, spray granulation methods, and crushing granulation methods, which can be used to produce uncoated tablets. Also, from a viewpoint of the granulation principles, granulation methods are largely divided into the dry granulation method and the wet granulation method. From a viewpoint of the stability of FTD and TPI, the dry granulation method is preferred.

According to the present invention, not adding the additives including the metal salt substantially can suppress increases in formation of related substances of FTD and TPI which are potentially formed when oral pharmaceutical compositions and pharmaceutical formulations comprising FTD and TPI as active ingredients are produced. The corresponding related substances herein mean components other than FTD, TPI, and additives, and mainly refer to structurally related compounds of the corresponding two active ingredients. Specifically, the related substances are substances other than FTD, TPI, and additives which are detected when measured in accordance with Liquid Chromatography described in the Japanese Pharmacopoeia, General Tests, Physical tests, after the oral pharmaceutical composition and pharmaceutical formulation of the present invention are stored under certain constant conditions.

Aspects and preferred embodiments of the present invention are shown below.

[1] An uncoated tablet comprising α,α,α-trifluorothymidine (FTD) and 5-chloro-6-(2-iminopyrrolidine-1-yl) methyl-2,4(1H,3H)-pyrimidine dione hydrochloride (TPI) as an active ingredient, comprising a sugar alcohol or a disaccharide as an excipient, one or more selected from the group consisting of hydrogenated oils and stearic acid as a lubricant, and being free of additives comprising a metal salt.
[2] The uncoated tablet according to [1], wherein the metal salt is selected from alkali metal salts and alkaline earth metal salts.
[3] The uncoated tablet composition according to any of [1] or [2], wherein the metal salt is selected from sodium salts, potassium salts, calcium salts, and magnesium salts.
[4] The uncoated tablet according to any of [1] to [3], wherein the content of the metal salt is from 0 to 0.1 parts by mass, preferably from 0 to 0.05 parts by mass, more preferably from 0 to 0.01 parts by mass, and still more preferably 0 parts by mass based on 1 part by mass of FTD.
[5] The uncoated tablet according to [1], wherein the sugar alcohol or disaccharide comprised as the excipient is one or more selected from lactose, sucrose, erythritol, and mannitol, and preferably one or more selected from lactose, sucrose, and mannitol.
[6] The uncoated tablet according to [1], wherein the content of the sugar alcohol or disaccharide is 3.6 parts by mass or more, preferably 3.6 to 50 parts by mass, more preferably 3.7 to 25 parts by mass, and still more preferably 3.7 to 10 parts by mass based on 1 part by mass of FTD.
[7] The uncoated tablet according to any of [1] to [6], further comprising, as a disintegrating agent, one or two selected from low-substituted hydroxypropyl cellulose, carmellose, corn starch, partly pregelatinized starch, and crospovidone, preferably one or more selected from low-substituted hydroxypropyl cellulose, carmellose, corn starch, and partly pregelatinized starch, and more preferably one or more selected from low-substituted hydroxypropyl cellulose, corn starch, and partly pregelatinized starch.
[8] The uncoated tablet according to [7], wherein a content of the disintegrating agent is from 2 to 16% by mass, preferably from 3 to 13% by mass, more preferably from 3 to 10% by mass, and particularly preferably from 3 to 7% by mass, in the total amount of the composition.
[9] The uncoated tablet according to [1], wherein the content of the lubricant is from 0.001 to 3% by mass and preferably from 0.01 to 2% by mass in the total composition.
[10] The uncoated tablet according to any of [1] to [9], comprising FTD and TPI at a molar ratio of 1:0.5.
[11] The uncoated tablet according to any of [1] to [10], wherein the oral pharmaceutical composition comprises FTD and TPI as active ingredients in a molar ratio of 1:0.5; comprises one or more selected from lactose, sucrose, or mannitol (preferably lactose) as the excipient; comprises one or more selected from low-substituted hydroxypropyl cellulose, corn starch, partly pregelatinized starch, and carmellose (preferably one or two selected from corn starch and partly pregelatinized starch) as the disintegrating agent; and one or two of hydrogenated oils and stearic acid (preferably stearic acid) as the lubricant.
[12] An uncoated tablet according to any of [1] to [11], wherein the tablet is coated.

### [Examples]

The present invention is described in more details hereinbelow referring to Examples, Comparative Examples, and Test Examples.

### Example 1

After 1 g of FTD, 0.471 g of TPI, 3.779 g of a lactose hydrate, 0.75 g of carmellose "NS-300" (manufactured by GOTOKU CHEMICAL COMPANY LTD.) and 0.15 g of stearic acid were sufficiently mixed in a mortar, 123 mg was weighed off and compression molded at 1 ton by use of a punch having a ϕ of 7 mm and an R of 10 mm and a hydraulic press (manufactured by RIKEN SEIKI CO., LTD) to thereby obtain tablets (see Table 1).

### Example 2

Tablets were obtained in accordance with the same method as in Example 1, except that carmellose was not added. However, the mass of the tablet was made to be 108 mg so as to reach a mass corresponding to 20 mg of FTD (see Table 1).

### Example 3

Tablets were obtained in accordance with the same method as in Example 1, except that corn starch "corn starch W" (manufactured by NIHON SHOKUHIN KAKO CO., LTD.) was used instead of carmellose (see Table 1).

### Example 4

Tablets were obtained in accordance with the same method as in Example 1, except that partly pregelatinized starch "PCS (PC-10)" (manufactured by Asahi Kasei Chemicals Corporation) was used instead of carmellose (see Table 1).

### Comparative Example 1

Tablets were obtained in accordance with the same method as in Example 1, except that carmellose calcium "E.C.G-505" (manufactured by GOTOKU CHEMICAL COMPANY LTD.) was used instead of carmellose (see Table 1).

### Comparative Example 2

Tablets were obtained in accordance with the same method as in Example 1, except that croscarmellose sodium "Ac-Di-Sol" (manufactured by Asahi Kasei Corporation) was used instead of carmellose (see Table 1).

### Test Example 1

Tablets obtained in Examples 1 to 4 and Comparative Examples 1 and 2 were stored at 40°C/75% R.H. for four weeks. Tablets stored at 5°C (in an airtight container) were also prepared for comparison.

The mass of the related substances formed was measured with Liquid Chromatography described in the Japanese Pharmacopoeia, General Tests, Physical tests. The results are shown in Table 1. It should be noted that peaks other than those of FTD, TPI, and additives are called related substance peaks and that the total mass of the related substances refers to the sum of the mass of the related substances calculated based on the area of the active ingredients from the area of the related substance peaks.

**[Table 1]**

| Unit: parts by mass | | | | | | | |
|---|---|---|---|---|---|---|---|
| | | Example | | | | Comparative Example | |
| | | 1 | 2 | 3 | 4 | 1 | 2 |
| FTD | | 20 | 20 | 20 | 20 | 20 | 20 |
| TPI | | 9.42 | 9.42 | 9.42 | 9.42 | 9.42 | 9.42 |
| Lactose hydrate | | 75.58 | 75.58 | 75.58 | 75.58 | 75.58 | 75.58 |
| Carmellose | | 15 | - | - | - | - | - |
| Corn starch | | - | - | 15 | - | - | - |
| Partly pregelatinized starch | | - | - | - | 15 | - | - |
| Carmellose calcium | | - | - | - | - | 15 | - |
| Croscarmellose sodium | | - | - | - | - | - | 15 |
| Stearic acid | | 3 | 3 | 3 | 3 | 3 | 3 |
| Total mass of the related substances (%) | Stored at 40°C/75% R.H. | 0.404 | 0.286 | 0.669 | 0.688 | 1.194 | 2.529 |
| | Stored at 5°C | 0.226 | 0.246 | 0.232 | 0.283 | 0.227 | 0.273 |

As clearly seen from the results of Table 1, Examples 1 to 4, which contain no additives comprising metal salts, had smaller increases in the total mass of the related substances compared to the tablets stored in the cool place at 5°C, even after stored under high-humidity conditions of 40°C/75% R.H. In contrast, noticeable increases in the total related substances were observed in Comparative Examples compared to the tablets stored in the cool place. From the above, in FTD and TPI-containing formulations, it was suggested that formulas containing metal salts as additives may induce increases in related substances.

### Example 5

In a stirring and mixing granulator (device name "Vertical Granulator VG-05", manufactured by Powrex Corporation), charged were 30 g of FTD, 14.13 g of TPI, 270.87 g of a lactose hydrate, and 45 g of carmellose, to which purified water/ethanol (1:1) mixture was added to thereby carry out granulation. The granulated product was dried using a fluid bed dryer (device name "FLOW-COATER MINI", manufactured by Freund Corporation), and then, screened with a sieve having an opening of 600 µm to thereby obtain a sized product.

After 2.4 g of this sized product and 0.6 g of stearic acid (manufactured by NOF CORPORATION) as a lubricant were mixed in a plastic bag, 150 mg was weighed off and compression molded at 1 ton by use of a punch having a ϕ of 7 mm and an R of 10 mm and a hydraulic press to thereby obtain tablets.

### Example 6

Tablets were obtained in accordance with the same method as in Example 5, except that stearic acid was not added. In this case, the mass of the tablet was made to be 120 mg so as to reach a mass corresponding to 10 mg of FTD.

### Example 7

Tablets were obtained in accordance with the same method as in Example 5, except that a hydrogenated castor oil "Lubriwax-101" (manufactured by Freund Corporation) was used instead of stearic acid.

### Comparative Example 3

Tablets were obtained in accordance with the same method as in Example 5, except that magnesium stearate (manufactured by TAIHEI CHEMICAL INDUSTRIAL CO., LTD.) was used instead of stearic acid.

### Test Example 2

In accordance with the method described in Test Example 1, tablets obtained in Examples 5 to 7 and Comparative Example 3 were stored at 60°C/80% R.H. for eight days. The mass of the related substances formed was measured with Liquid Chromatography described in the Japanese Pharmacopoeia, General Tests, Physical tests. The results are shown in Table 2.

**[Table 2]**

| Unit: parts by mass | | | | |
|---|---|---|---|---|
| | Example | | | Comparative Example |
| | 5 | 6 | 7 | 3 |
| FTD | 10 | 10 | 10 | 10 |
| TPI | 4.71 | 4.71 | 4.71 | 4.71 |
| Lactose hydrate | 90.29 | 90.29 | 90.29 | 90.29 |
| Carmellose | 15 | 15 | 15 | 15 |
| Stearic acid | 30 | - | - | - |
| Hydrogenated castor oil | - | - | 30 | - |
| Magnesium stearate | - | - | - | 30 |
| Total mass of the related substances (%) Stored at 60°C/80% R.H. | 6.4 | 6.4 | 6.3 | 38.1 |

As clearly seen from the results of Table 2, also in the case where a lubricant was added, Examples 5 and 7, which do not contain lubricants comprising metal salts, had small increases in the total mass of the related substances even after stored under very severe high-temperature and high-humidity conditions of 60°C/80% R.H., as Example 6, which contains no lubricant. In contrast, noticeable increases in the total related substances were observed in Comparative Example 3.

From the results above-described, it was found that FTD and TPI-containing formulations which have high stability even under very severe high-temperature and/or high-humidity conditions can be obtained by not adding additives comprising metal salts.

### Example 8

In a plastic bag, 400 g of FTD, 188.4 g of TPI, 1511.6 g of a lactose hydrate, 300 g of carmellose, and 40 g of stearic acid were mixed. This mixture was tableted with a rotary tableting machine into tablets having a diameter of 15 mm and a mass of 800 mg. Then, the tablets were crushed with a crusher to there by obtain a granulated product. To 122 parts of this granulated product, 1 part of stearic acid was further added and mixed in a plastic bag. Uncoated tablets having a diameter of 7 mm and a mass of 123 mg were obtained by use of a rotary tableting machine (see Table 3) .

### Example 9 (Reference)

In a mortar, 1 g of a mixture of 1 part of FTD and 0.471 parts of TPI, 6 g of a lactose hydrate, and 1 g of carmellose were mixed. From this mixture, uncoated tablets having a mass of 235.36 mg were obtained by use of a hydraulic press (see Table 3).

### Example 10

In a plastic bag, 1200 g of FTD, 565.2 g of TPI, 7258.8 g of a lactose hydrate, 480 g of partly pregelatinized starch, and 96 g of stearic acid were mixed. From this mixture, uncoated tables having a diameter of 7 mm and a mass of 120 mg were obtained by use of a rotary tableting machine (see Table 3).

### Example 11

In accordance with the method described in Example 10, 100 g of FTD, 47.1 g of TPI, 371.9 g of a lactose hydrate, 100 g of partly pregelatinized starch, and 6 g of stearic acid were mixed in a plastic bag. From this mixture, uncoated tablets having a diameter of 7 mm and a mass of 125 mg were obtained by use of a rotary tableting machine (see Table 4).

### Example 12

In accordance with the method described in Example 10, 100 g of FTD, 47.1 g of TPI, 371.9 g of a lactose hydrate, 25 g of partly pregelatinized starch, and 6 g of stearic acid were mixed in a plastic bag. From this mixture, uncoated tablets having a diameter of 7 mm and a mass of 110 mg were obtained by use of a rotary tableting machine (see Table 4).

### Example 13

In according with the method described in Example 10, 100 g of FTD, 47.1 g of TPI, 371.9 g of a lactose hydrate, 50 g of partly pregelatinized starch, and 6 g of stearic acid were mixed in a plastic bag. From this mixture, uncoated tablets having a diameter of 7 mm and a mass of 115 mg were obtained by use of a rotary tableting machine (see Table 4).

### Example 14

In accordance with the method described in Example 10, 100 g of FTD, 47.1 g of TPI, 521.9 g of a lactose hydrate, 75 g of partly pregelatinized starch, and 6 g of stearic acid were mixed in a plastic bag. From this mixture, uncoated tablets having a diameter of 7 mm and a mass of 150 mg were obtained by use of a rotary tableting machine (see Table 4).

### Example 15

In accordance with the method described in Example 10, 100 g of FTD, 47.1 g of TPI, 671.9 g of a lactose hydrate, 75 g of partly pregelatinized starch, and 6 g of stearic acid were mixed in a plastic bag. From this mixture, uncoated tablets having a diameter of 7 mm and a mass of 150 mg were obtained by use of a rotary tableting machine (see Table 4).

**[Table 3]**

| Unit: parts by mass | | | |
|---|---|---|---|
| | Example | | |
| | 8 | 9 | 10 |
| FTD | 1 | 1 | 1 |
| TPI | 0.47 | 0.47 | 0.47 |
| Lactose hydrate | 3.78 | 8.83 | 6.05 |
| Carmellose | 0.75 | 1.47 | - |
| Partly pregelatinized starch | - | | 0.4 |
| Stearic acid | 0.15 | - | 0.08 |
| Total | 6.15 | 11.77 | 8 |

**[Table 4]**

| Unit: parts by mass | | | | | |
|---|---|---|---|---|---|
| | Example | | | | |
| | 11 | 12 | 13 | 14 | 15 |
| FTD | 1 | 1 | 1 | 1 | 1 |
| TPI | 0.471 | 0.471 | 0.471 | 0.471 | 0.471 |
| Lactose hydrate | 3.719 | 3.719 | 3.719 | 5.219 | 6.719 |
| Partly pregelatinized starch | 1 | 0.25 | 0.5 | 0.75 | 0.75 |
| Stearic acid | 0.06 | 0.06 | 0.06 | 0.06 | 0.06 |
| Total | 6.25 | 5.5 | 5.75 | 7.5 | 9 |

### Example 16

In accordance with the method described in Example 10, 50 g of FTD, 23.55 g of TPI, 226.45 g of a lactose hydrate, and 3 g of stearic acid were mixed in a plastic bag. From this mixture, uncoated tablets having a mass of 121.2 mg were obtained by use of a rotary tableting machine (see Table 5).

### Example 17

In accordance with the method described in Example 10, 50 g of FTD, 23.55 g of TPI, 211.45 g of a lactose hydrate, 15 g of a disintegrating agent (any of corn starch , partly pregelatinized starch, or low-substituted hydroxypropyl cellulose), and 3 g of stearic acid were mixed in a plastic bag. From this mixture, uncoated tablets having a mass of 121.2 mg were obtained by use of a rotary tableting machine (see Table 5).

### Example 18

In accordance with the method described in Example 17, 50 g of FTD, 23.55 g of TPI, 196.45 g of a lactose hydrate, 30 g of a disintegrating agent (any of corn starch, partly pregelatinized starch or low-substituted hydroxypropyl cellulose), and 3 g of stearic acid were mixed in a plastic bag. From this mixture, uncoated tablets having a mass of 121.2 mg were obtained by use of a rotary tableting machine (see Table 5).

### Test Example 3

In accordance with the method described in Test Example 1, tablets obtained in Examples 16, 17, and 18 were stored at 40°C/75% R.H. in open conditions for two weeks, and then, the total mass of the related substances was measured (see Table 5).

As the result, noticeable increases in related substances were not observed in any of the tablets.

**[Table 5]**

| Unit: parts by mass | | | | | | | |
|---|---|---|---|---|---|---|---|
| | Example | | | | | | |
| | 16 | 17 | | | 18 | | |
| FTD | 1 | 1 | 1 | 1 | 1 | 1 | 1 |
| TPI | 0.471 | 0.471 | 0.471 | 0.471 | 0.471 | 0.471 | 0.471 |
| Lactose hydrate | 4.529 | 4.229 | 4.229 | 4.229 | 3.929 | 3.929 | 3.929 |
| Corn starch | - | 0.3 | - | - | 0.6 | - | - |
| Partly pregelatinized starch | - | - | 0.3 | - | - | 0.6 | - |
| Low-substituted hydroxypropyl cellulose | - | - | - | 0.3 | - | - | 0.6 |
| Stearic acid | 0.06 | 0.06 | 0.06 | 0.06 | 0.06 | 0.06 | 0.06 |
| Total | 6.06 | 6.06 | 6.06 | 6.06 | 6.06 | 6.06 | 6.06 |
| Total mass of the related substances (%) | 0.188 | 0.2 | 0.266 | 0.332 | 0.282 | 0.334 | 0.391 |

## Claims

1. An uncoated tablet comprising α,α,α-trifluorothymidine and 5-chloro-6-(2-iminopyrrolidine-1-yl)methyl-2,4(1H,3H)-pyrimidine dione hydrochloride as an active ingredient; comprising a sugar alcohol or a disaccharide as an excipient, one or more selected from the group consisting of hydrogenated oils and stearic acid as a lubricant, and being free of an additive comprising a metal salt.

2. The uncoated tablet according to claim 1, wherein the metal salt is selected from alkali metal salts and alkaline earth metal salts.

3. The uncoated tablet according to claim 1 or 2, wherein the metal salt is selected from sodium salts, potassium salts, calcium salts, and magnesium salts.

4. The uncoated tablet according to claim 1, wherein the sugar alcohol or disaccharide comprised as the excipient are one or more selected from lactose, sucrose, erythritol, and mannitol.

5. The uncoated tablet according to claim 4, wherein the content of the sugar alcohol or disaccharide is 3.6 parts by mass or more based on 1 part by mass of α,α,α-trifluorothymidine.

6. The uncoated tablet according to any of claims 1 to 5, further comprising one or more selected from low-substituted hydroxypropyl cellulose, corn starch, partly pregelatinized starch, carmellose, and crospovidone as a disintegrating agent.

7. The uncoated tablet according to claim 6, wherein the content of the disintegrating agent is from 2 to 16% by mass in the total amount of the composition.

8. The uncoated tablet according to claim 1, wherein the content of the lubricant is from 0.001 to 3% by mass in the total composition.

9. The uncoated tablet according to any of claims 1 to 8, comprising α,α,α-trifluorothymidine and 5-chloro-6-(2-iminopyrrolidine-1-yl)methyl-2,4(1H,3H)-pyrimidine dione hydrochloride at a molar ratio of 1:0.5.

10. The uncoated tablet according to any of claims 1 to 9, wherein the uncoated tablet comprises α,α,α-trifluorothymidine and 5-chloro-6-(2-iminopyrrolidine-1-yl)methyl-2,4(1H,3H)-pyrimidine dione hydrochloride as the active ingredients in a molar ratio of 1:0.5; comprises one or more selected from lactose, sucrose, or mannitol as the excipient; and one or more selected from low-substituted hydroxypropyl cellulose, corn starch, partly pregelatinized starch, and carmellose as the disintegrating agent.

11. An oral pharmaceutical formulation comprising the uncoated tablet according to any of claims 1 to 10, wherein the tablet is coated.

12. The coated tablet according to claim 11, wherein the tablet comprises magnesium stearate on the surface.

13. A method for stabilizing an uncoated tablet comprising α,α,α-trifluorothymidine and 5-chloro-6-(2-iminopyrrolidine-1-yl)methyl-2,4(1H,3H)-pyrimidine dione hydrochloride as an active ingredient; comprising a sugar alcohol or a disaccharide as an excipient, and one or more selected from the group consisting of hydrogenated oils and stearic acid as a lubricant, **characterized by** adding no additive comprising a metal salt.

## Patentansprüche

1. Unbeschichtete Tablette, die α,α,α-Trifluorthymidin und 5-Chlor-6-(2-iminopyrrolidin-1-yl)methyl-2,4(1H,3H)-pyrimidindion-Hydrochlorid als Wirkstoff umfasst; die einen Zuckeralkohol oder ein Disaccharid als Hilfsstoff, und eines oder mehrere ausgewählt aus der Gruppe bestehend aus hydrierten Ölen und Stearinsäure als Schmiermittel umfasst, und die frei von einem Additiv ist, das ein Metallsalz enthält.

2. Die unbeschichtete Tablette nach Anspruch 1, wobei das Metallsalz ausgewählt ist aus Alkalimetallsalzen und Erdalkalimetallsalzen.

3. Die unbeschichtete Tablette nach Anspruch 1 oder 2, wobei das Metallsalz ausgewählt ist aus Natriumsalzen, Kaliumsalzen, Calciumsalzen und Magnesiumsalzen.

4. Die unbeschichtete Tablette nach Anspruch 1, wobei der Zuckeralkohol oder das Disaccharid, das als Hilfsstoff umfasst ist, eines oder mehrere sind ausgewählt aus Lactose, Saccharose, Erythritol und Mannitol.

5. Die unbeschichtete Tablette nach Anspruch 4, wobei der Gehalt an Zuckeralkohol oder Disaccharid 3,6 Massenteile oder mehr basierend auf 1 Massenteil α,α,α-Trifluorthymidin beträgt.

6. Die unbeschichtete Tablette nach einem der Ansprüche 1 bis 5, ferner umfassend eines oder mehrere ausgewählt aus niedrig-substituierter Hydroxypropylcellulose, Maisstärke, teilweise vorgelatinierter Stärke, Carmellose und Crospovidon als Sprengmittel.

7. Die unbeschichtete Tablette nach Anspruch 6, wobei der Gehalt an Sprengmittel von 2 bis 16 Masseprozent in der Gesamtmenge der Zusammensetzung beträgt.

8. Die unbeschichtete Tablette nach Anspruch 1, wobei der Gehalt an Schmiermittel von 0,001 bis 3 Masseprozent in der gesamten Zusammensetzung beträgt.

9. Die unbeschichtete Tablette nach einem der Ansprüche 1 bis 8, die α,α,α-Trifluorthymidin und 5-Chlor-6-(2-iminopyrrolidin-1-yl)methyl-2,4(1H,3H)-pyrimidindion-Hydrochlorid in einem Molverhältnis von 1:0,5 umfasst.

10. Die unbeschichtete Tablette nach einem der Ansprüche 1 bis 9, wobei die unbeschichtete Tablette a,a,a-Trifluorthymidin und 5-Chlor-6-(2-iminopyrrolidin-1-yl)methyl-2,4(1H,3H)-pyrimidindion-Hydrochlorid als Wirkstoffe in einem Molverhältnis von 1:0,5 umfasst; die eines oder mehrere ausgewählt aus Lactose, Saccharose oder Mannitol als Hilfsstoff; und eines oder mehrere ausgewählt aus niedrig-substituierter Hydroxypropylcellulose, Maisstärke, teilweise vorgelatinierte Stärke und Carmellose als Sprengmittel umfasst.

11. Orale pharmazeutische Formulierung, umfassend die unbeschichtete Tablette nach einem der Ansprüche 1 bis 10, wobei die Tablette beschichtet ist.

12. Die beschichtete Tablette nach Anspruch 11, wobei die Tablette Magnesiumstearat auf ihrer Oberfläche umfasst.

13. Verfahren zur Stabilisierung einer unbeschichteten Tablette, die a,a,a-Trifluorthymidin und 5-Chlor-6-(2-iminopyrrolidin-1-yl)methyl-2,4(1H,3H)-pyrimidindion-Hydrochlorid als Wirkstoff umfasst; die einen Zuckeralkohol oder ein Disaccharid als Hilfsstoff und eines oder mehrere ausgewählt aus der Gruppe bestehend aus hydrierten Ölen und Stearinsäure als Schmiermittel umfasst, **dadurch gekennzeichnet, dass** kein Additiv zugegeben wird, das ein Metallsalz umfasst.

## Revendications

1. Comprimé non enrobé comprenant de l'α,α,α-trifluorothymidine et du chlorhydrate de 5-chloro-6-(2-iminopyrrolidin-1-yl)méthyl-2,4(1H,3H)-pyrimidinedione en tant que principe actif ; comprenant un alcool de sucre ou un disaccharide en tant qu'excipient, un ou plusieurs choisis dans le groupe constitué par les huiles hydrogénées et l'acide stéarique en tant que lubrifiant, et exempt d'un additif comprenant un sel métallique.

2. Comprimé non enrobé selon la revendication 1, dans lequel le sel métallique est choisi parmi les sels de métal alcalin et les sels de métal alcalino-terreux.

3. Comprimé non enrobé selon la revendication 1 ou 2, dans lequel le sel métallique est choisi parmi les sels de sodium, les sels de potassium, les sels de calcium, et les sels de magnésium.

4. Comprimé non enrobé selon la revendication 1, dans lequel l'alcool de sucre ou le disaccharide compris en tant qu'excipient est un ou plusieurs choisis parmi le lactose, le saccharose, l'érythritol et le mannitol.

5. Comprimé non enrobé selon la revendication 4, dans lequel la teneur en l'alcool de sucre ou le disaccharide est de 3,6 parties en masse ou plus basées sur 1 partie en masse d'α,α,α-trifluorothymidine.

6. Comprimé non enrobé selon l'une quelconque des revendications 1 à 5, comprenant en outre un ou plusieurs choisis parmi l'hydroxypropylcellulose faiblement substituée, l'amidon de maïs, l'amidon partiellement pré-gélatinisé, la carmellose, et la crospovidone en tant qu'agent délitant.

7. Comprimé non enrobé selon la revendication 6, dans lequel la teneur en l'agent délitant est de 2 à 16 % en masse de la quantité totale de la composition.

8. Comprimé non enrobé selon la revendication 1, dans lequel la teneur en lubrifiant est de 0,001 à 3 % en masse de la composition totale.

9. Comprimé non enrobé selon l'une quelconque des revendications 1 à 8, comprenant de l'α,α,α-trifluorothymidine et du chlorhydrate de 5-chloro-6-(2-iminopyrrolidin-1-yl)méthyl-2,4(1H,3H)-pyrimidinedione en un rapport molaire de 1/0,5.

10. Comprimé non enrobé selon l'une quelconque des revendications 1 à 9, dans lequel le comprimé non enrobé comprend de l'α,α,α-trifluorothymidine et du chlorhydrate de 5-chloro-6-(2-iminopyrrolidin-1-yl)méthyl-2,4(1H,3H)-pyrimidinedione en tant que principes actifs en un rapport molaire de 1/0,5 ; comprend un ou plusieurs choisis parmi le lactose, le saccharose et le mannitol en tant qu'excipient ; et un ou plusieurs choisis parmi l'hydroxypropylcellulose faiblement substituée, l'amidon de maïs, l'amidon partiellement pré-gélatinisé et la carmellose en tant qu'agent délitant.

11. Formulation pharmaceutique orale comprenant le comprimé non enrobé selon l'une quelconque des revendications 1 à 10, dans laquelle le comprimé est enrobé.

12. Comprimé enrobé selon la revendication 11, dans lequel le comprimé comprend du stéarate de magnésium sur sa surface.

13. Procédé pour stabiliser un comprimé non enrobé comprenant de l'α,α,α-trifluorothymidine et du chlorhydrate de 5-chloro-6-(2-iminopyrrolidin-1-yl)méthyl-2,4(1H,3H)-pyrimidinedione en tant que principe actif ; comprenant un alcool de sucre ou un disaccharide en tant qu'excipient, un ou plusieurs choisis dans le groupe constitué par les huiles hydrogénées et l'acide stéarique en tant que lubrifiant, **caractérisé par** l'addition d'aucun additif comprenant un sel métallique.
